# EUROPÄISCHE PATENTSCHRIFT

(11) **EP 1 560 501 B1**
(45) Veröffentlichungstag und Bekanntmachung des Hinweises auf die Patenterteilung: **02.06.2010**
(21) Anmeldenummer: 03757954.7
(22) Anmeldetag: 10.10.2003
(51) Int. Cl.: A23J 3/14, A23J 3/16, A23G 9/38, A23L 1/03, A23L 1/20, A23L 1/305, A23L 1/23, A23C 20/02

(54) **BIOTECHNOLOGISCH ERZEUGBARES PROTEINHALTIGES PRÄPARAT, VERFAHREN ZU SEINER HERSTELLUNG UND SEINE VERWENDUNG ALS LEBENSMITTELZUTAT**
PROTEIN-CONTAINING PREPARATION WHICH CAN BE BIOTECHNOLOGICALLY PRODUCED, METHOD FOR THE PRODUCTION THEREOF, AND USE OF THE SAME AS A FOOD INGREDIENT
PREPARATION BIOTECHNOLOGIQUE CONTENANT DES PROTEINES, PROCEDE DE FABRICATION ASSOCIE ET UTILISATION COMME COMPLEMENT ALIMENTAIRE

(30) Priorität: 16.10.2002 DE 10248263; 25.10.2002 EP 02023980
(43) Veröffentlichungstag der Anmeldung: 10.08.2005
(73) Patentinhaber: Fraunhofer-Gesellschaft zur Förderung der angewandten Forschung e.V., 80686 München (DE)
(72) Erfinder: BACK, Werner, 85354 Freising (DE); BOENISCH, Martin, 81539 München (DE); MUELLER, Klaus, 85356 Freising (DE); BEZ, Jürgen, 80469 München (DE); WAESCHE, Andreas, 85638 Moosburg (DE)
(74) Vertreter: Olgemöller, Luitgard Maria
(86) Internationale Anmeldenummer: PCT/EP2003/011264
(87) Internationale Veröffentlichungsnummer: WO 2004/034806

(56) Entgegenhaltungen:
- EP-A- 0 006 380
- EP-A- 0 988 793
- WO-A-02/05657
- DD-A- 278 058
- GB-A- 1 356 363
- US-A- 4 678 673
- DATABASE FSTA [Online] INTERNATIONAL FOOD INFORMATION SERVICE (IFIS), FRANFURT/MAIN, DE; MOK C ET AL: "Risogurt, a mixture of lactic acid fermented rice and soybean protein: development and properties." Database accession no. 92-1-08-m0027 XP002227512 & KOREAN JOURNAL OF FOOD SCIENCE AND TECHNOLOGY 1991 KOREA FOOD RES. INST., SAN 46-1 BAEKHYONDONG BOONDANGKU, SONGNAM, KYONGKIDO 462-420, KOREA REPUBLIC, Bd. 23, Nr. 6, Seiten 745-749,
- DATABASE FSTA [Online] INTERNATIONAL FOOD INFORMATION SERVICE (IFIS), FRANFURT/MAIN, DE; PAIK I S ET AL: "Keeping quality of yogurt beverage prepared from soy protein concentrate." Database accession no. 85-2-11-h0079 XP002227513 & KOREAN JOURNAL OF FOOD SCIENCE AND TECHNOLOGY 1985 DEP. OF FOODS & NUTR., DUKSUNG WOMEN'S COLL., SEOUL, S. KOREA, Bd. 17, Nr. 1, Seiten 45-50,
- ANKENMAN GRANATA L ET AL: "IMPROVED ACID FLAVOR AND VOLATILE COMPOUND PRODUCTION IN A HIGH PROTEIN AND FIBER SOYMILK YOGHURT-LIKE PRODUCT" JOURNAL OF FOOD SCIENCE, INSTITUTE OF FOOD TECHNOLOGISTS. CHICAGO, US, Bd. 61, Nr. 2, 1. März 1996 (1996-03-01), Seiten 331-336, XP000589501 ISSN: 0022-1147 in der Anmeldung erwähnt
- CHENG Y J ET AL: "SOGURT, A YOGURT-LIKE SOYBEAN PRODUCT: DEVELOPMENT AND PROPERTIES" JOURNAL OF FOOD SCIENCE, INSTITUTE OF FOOD TECHNOLOGISTS. CHICAGO, US, Bd. 55, Nr. 4, Juli 1990 (1990-07), Seiten 1178-1179, XP000159987 ISSN: 0022-1147 in der Anmeldung erwähnt
- CAMACHO L ET AL: "NUTRITIONAL QUALITY OF LUPINE (LUPINUS ALBUS CV. MULTOLUPA) AS AFFECTED BY LACTIC ACID FERMENTATION" INTERNATIONAL JOURNAL OF FOOD MICROBIOLOGY, ELSEVIER SCIENCE PUBLISHERS, AMSTERDAM, NL, Bd. 14, Nr. 3/4, 1991, Seiten 277-286, XP000986834 ISSN: 0168-1605 in der Anmeldung erwähnt
- MITAL B K ET AL: "FLAVOR ACCEPTABILITY OF UNFERMENTED AND LACTIC-FERMENTED SOY MILKS" JOURNAL OF MILK AND FOOD TECHNOLOGY, THE ASSOCIATION, AMES, IA, US, Bd. 5, Nr. 39, Mai 1976 (1976-05), Seiten 342-344, XP008005528 ISSN: 0022-2747
- DATABASE FSTA [Online] INTERNATIONAL FOOD INFORMATION SERVICE (IFIS), FRANFURT/MAIN, DE; HAN O ET AL: "Lactic acid fermentation of lupinseed milk." Database accession no. 87-1-05-h0099 XP002227514 & KOREAN JOURNAL OF APPLIED MICROBIOLOGY AND BIOENGINEERING 1985 DEP. OF FOOD TECH., KOREA UNIV., SEOUL, KOREA REPUBLIC, Bd. 13, Nr. 3, Seiten 191-198,
- CZARNECKA M ET AL: "EFFECT OF LACTIC FERMENTATION AND EXTRUSION OF BEAN AND PEA SEEDS ON NUTRITIONAL AND FUNCTIONAL PROPERTIES" DIE NAHRUNG, VCH VERLAGSGESELLSCHAFT, WEINHEIM, XX, Bd. 42, Nr. 1, 1998, Seiten 7-11, XP008012511 ISSN: 0027-769X
- PINTHONG R ET AL: "The development of a soya-based yoghurt. I. Acid production by lactic acid bacteria." JOURNAL OF FOOD TECHNOLOGY 1980 DEP. OF FOOD SCI., UNIV. OF READING, LONDON ROAD, READING RG1 5AQ, UK, Bd. 15, Nr. 6, Seiten 647-652, XP008012461 in der Anmeldung erwähnt
- HAGEN M ET AL: "PRODUCTION OF ICE CREAM CONTAINING PROBIOTIC BACTERIA" MILCHWISSENSCHAFT, VV GMBH VOLKSWIRTSCHAFTLICHER VERLAG. MUNCHEN, DE, Bd. 54, Nr. 5, 1999, Seiten 265-267, XP000852082 ISSN: 0026-3788

## Beschreibung

Die vorliegende Erfindung betrifft ein proteinhaltiges Präparat pflanzlichen Ursprungs mit deutlich verbesserten sensorischen und ggf. auch ernährungsphysiologischen sowie unvermindert guten technofunktionellen Eigenschaften, das durch Fermentation mit einem Milchsäurebakterium gewonnen werden kann. Dieses Präparat eignet sich als vielseitig verwendbare Lebensmittelzutat.

Proteine bzw. proteinhaltige Präparate werden als Zutaten für die Lebensmittel- und Futtermittelindustrie genutzt und finden vielfach Anwendung in der Formulierung von Lebensmitteln (Fleisch- und Wurstwaren, Backwaren, Feinkostprodukten, Getränken, Eiscreme und vielerlei mehr). Die große Bedeutung von Proteinprodukten liegt zunächst in der Versorgung von Menschen mit essentiellen Aminosäuren. Darüber hinaus bieten proteinhaltige Präparate einen mehrfachen Nutzen, da sie aufgrund ihrer technischen Eigenschaften ("Technofunktionalität") auch zur Verbesserung oder Kontrolle einer Vielzahl von Eigenschaften wie Wasser- oder Ölbindung, Schaumbildung, Texturierung, Dispergierbarkeit, Viskositätskontrolle oder Emulgierbarkeit oder dgl. eingesetzt werden können.

Je nach Art, Herstellungsweise und Herkunft verfügen proteinhaltige Präparate über unterschiedliche Eigenschaftsmerkmale hinsichtlich der technofunktionellen Wirkung in Rezepturen, aber auch hinsichtlich deren sensorischer Beeinflussung.

Häufig divergiert die Einsatzkonzentration pflanzlicher Proteinpräparate zu Gunsten von Proteinpräparaten tierischer Herkunft (Gelatine, Milchprotein, Milchpulver, Molkenpulver), weil pflanzliche Proteinpräparate über herkunftsbezogene Begleitsubstanzen verfügen, die vom ernährungsphysiologischen Standpunkt aus unerwünscht sind und/oder den sensorischen und organoleptischen Eindruck der Rezeptur nachteilig beeinflussen. Proteinpräparate pflanzlichen Ursprungs sind nämlich in der Regel von Fehlaromen begleitet, die als "bohnig" beschrieben werden können und wahrscheinlich von Aldehyden wie Hexanal herrühren, und sie enthalten meist antinutritiv wirkende Substanzen wie Trypsininhibitoren und/oder unverdaubare Materialien wie z.B. a-1,6-glykosidisch verknüpfte Kohlenhydrate, die Flatulenz verursachen können (siehe P. Scalabrini et al., Int. J. of Food Microbiology 39, 213-219 (1998)).

Es ist bekannt, dass der sensorische Eindruck und der Nährwert von Proteinpräparaten durch eine biotechnologische Behandlung dieser Präparate verändert und verbessert werden kann. So weiß man beispielsweise, dass sich der Anteil antinutritiv wirkender oder unverdaubarer Substanzen in Proteinpräparaten aus Sojabohnen durch Fermentation reduzieren lässt. Wie von H.L. Wang et al. in J. Milk Food Technol, 37 71 (1974) gefunden wurde, wächst *Lactobacillus acidophilus* in Sojamilch ohne Zusatz von Zucker. Die Lactobacillen setzen also die dort ausschließliche vorhandenen α-glykosidisch verknüpften Kohlenhydrate als alleinige Energiequelle um. Matteuzzi et al., Int. J. of Food Microbiol., 39 (1998), 213-219, und H.L. Wang (a.a.O.) konnten zeigen, dass Bifidobacteriumstämme unerwünschte Aromakomponenten, die aus dem oxidativen Abbau von ungesättigten Fettsäuren durch Lipoxygenase-Aktivität gebildet werden, abbauen können. E.R. Bucker et al. beschreiben in Journal of Food Science, 44, 1534 (1979) die Fermentation von Erdnussmilch mit 19 verschiedenen MilchsäureBakterien unter Milchsäureproduktion. Von L.R. Beuchat et al., Journal of Food Science, 43, 1109 (1978) wurde festgestellt, dass die sensorischen Eigenschaften einer derart fermentierten Milch weniger unangenehm sind, so dass sie bei der Weiterverarbeitung in Gebäckwaren mit Buttermilch konkurrieren können. Allerdings wurden die sensorischen Eigenschaften nur indirekt bewertet, da ausschließlich mit Fruchtaromen versetzte Gebäckstücke getestet wurden. L. Camacho et al. untersuchten in Int. J. of Food Microbiology 14, 277-286 (1991) die Fermentation eines Lupinenbohnenextrakts mit verschiedenen Mikroorganismen der Gattung Lactobacillus und deren Auswirkung auf den Gehalt an Alkaloiden, Lactoflavin und einigen Aminosäuren und damit auf den ernährungsphysiologischen Wert der so erhaltenen Lupinenmilch. L. Ankenman Granata et al., J. of Food Science 61, 33 (1996) stellten fest, dass sich geschmackliche Leitsubstanzen wie Diacetyl in fermentierten Sojamilch-Produkten in vergleichbaren Konzentrationen finden lassen wie in einer Kontrolle aus (tierischer) Milch, wenn diesen Produkten Caseinat, Casein-Hydrolysat und Molkeprotein-Hydrolysat zugegeben worden war.

I.S. Paik et al. beschreiben in Korean Journal of Food Science Technology 17(1), Seiten 45 bis 50 (1985) die Bereitung eines Soja-Joghurt-Getränks, das durch Fermentation eines Sojaprotein-Konzentrats mit Hilfe von Lactobacillus acidophilus hergestellt wurde. Die Eigenschaften eines mit Milchsäurebakterien fermentierten Sojaproteins aus einem Sojaprotein-Isolat wurden von C. Mok et al. untersucht, wie in Korean Journal of Food Science Technology 23(6), Seiten 745 bis 749 (1991) beschrieben. Dabei stellte sich heraus, dass das fermentierte Protein-Isolat schlechtere Geruchs- und Geschmackseigenschaften besaß als mit Milchsäurebakterien fermentierter Reis.

Ausgangspunkt für die biotechnologische Behandlung von Proteinpräparaten aus geeigneten Pflanzen(teilen) ist in der Regel eine durch Extraktion der ganzen Pflanzen(teile), z.B. der Saaten oder Bohnen, gewonnene "Milch". Dabei werden üblicherweise die Ausgangsmaterialien in Wasser gequollen, ggf. vorbehandelt, z.B. mit Natriumbicarbonat, ggf. blanchiert und/oder vermahlen und dann extrahiert. Die erhaltene Milch wird filtriert und dann pasteurisiert oder gekocht. Für Sojamilch wird oft eine Variante des sogenannten Illinois-Prozesses angewandt (siehe z.B. A.I. Nelson et al., J. of Food Science 41 (1976), 57 oder K.M. Kamaly, Food Research Int., 30, (1997) 675-682. Ebenfalls beschrieben sind Produktionsbeispiele, die aus Lupinen- oder Sojamilch unter Zuhilfenahme von geeigneten Milchsäurebakterien in biotechnologischen Verfahren ein joghurtähnliches Produkt erzeugen. Hierbei spielen neben den nutritiven und sensorischen vor allem die rheologischen Eigenschaften des biotechnologischen Erzeignisses eine entscheidende Rolle (siehe R. Pinthong et al., J. Fd Technol. 15 (1980), 647-652). Die Sojamilch wird nach Pinthong, a.a.O., mit Milchsäurebakterien/Joghurtstarterkulturen (*Lactobacillus bulgaricus, Streptococcus thermophilus*) fermentiert. Dabei entstehen in fermentierter Sojamilch Fehlaromen, die auf die Lipoxygenase-Aktivität und die Freisetzung von Leitsubstanzen für Ranzigkeit zurückführbar sind. Y.J. Cheng et al. fanden dagegen in Journal of Food Science, 55 (1990), 1178, dass ein Produkt, das durch Fermentation von Sojamilch bei Zusatz von Lactose und Milchproteinen und Joghurtstarterkulturen entsteht ("Sogurt"), rheologisch und sensorisch akzeptabel ist. Es ließe sich weitere Literatur anführen, in der teils die guten technischen und sensorischen Eigenschaften der milchartigen Produkte gelobt, teils deren Fehlen bemängelt wird. Die Bewertung der Sensorik beruht dabei in der Regel auf subjektiven Tests.

Aufgabe der vorliegenden Erfindung ist es, ein proteinhaltiges Präparat aus pflanzlichen Materialien bereitzustellen, das so verändert wurde, dass es zuverlässig positive Geschmacks- und Geruchseigenschaften aufweist, die ansonsten Milchprodukten zugeordnet werden, und dessen technische Eigenschaften wie Emulgieraktivität, Gelbildung und Schaumbildungseigenschaften gegenüber denen des Ausgangsmaterials nicht oder nicht wesentlich verschlechtert sind.

Überraschend konnte gefunden werden, dass ein solches Präparat erhältlich ist, wenn man pflanzliche Ausgangsmaterialien, deren Proteingehalt, bezogen auf die Trockensubstanz, bei mindestens 60 Gew.-%, vorzugsweise bei mindestens 70 Gew.-%, und noch stärker bevorzugt bei mindestens 80 Gew.-% und maximal bei 100 Gew.-%, bevorzugt bei 99 Gew.-% und stärker bevorzugt bei 90 bis 95 Gew.-% liegt, biotechnologisch behandelt, insbesondere wenn man sie mit Milchsäure produzierenden Mikroorganismen fermentiert, sofern die pflanzlichen Ausgangsmaterialien Lupinensamen enthalten oder daraus bestehen.

Der Ausdruck "Proteingehalt", wie er in der vorliegenden Anmeldung verwendet wird, ist definiert als der Gehalt, der sich aus der Bestimmung des Stickstoff und dessen Multiplikation mit dem Faktor 6,25 errechnet.

Durch die biotechnologische Behandlung (Fermentation) der Ausgangsmaterialien entsteht Milchsäure, und deshalb enthält das erfindungsgemäße proteinhaltige Präparat Milchsäure. Je nach Art der eingesetzten Milchsäurebakterien handelt es sich dabei um D-Milchsäure, um L-Milchsäure oder um eine Mischung aus den beiden optischen Varianten. Bekanntlich ist die linksdrehende L-Milchsäure ernährungsphysiologisch besonders wertvoll, und deshalb ist es bevorzugt, dass ein großer Teil oder die gesamte Milchsäure als L-Milchsäure vorliegt. Wünschenswert ist es weiterhin, dass das erfindungsgemäße Proteinpräparat eine größere Menge an Milchsäure enthält, und zwar vorzugsweise mindestens ca. 5g/l und stärker bevorzugt mindestens ca. 8g/l. In besonders günstigen Fällen kann man sogar 10g/l Milchsäure oder noch mehr erhalten, wie unten näher dargelegt wird.

Weiterhin ist das erfindungsgemäße proteinhaltige Präparat gekennzeichnet durch ein milchproduktartiges Aroma. Das Aroma entsteht durch die Fermentation. Eine Leitsubstanz dieses Aromas ist Diacetyl, und in vielen Fällen ist ein hoher Diacetylgehalt wünschenswert, insbesondere, wenn das Proteinpräparat als Zutat für Lebensmittelzubereitungen eingesetzt werden soll, weil der Geschmack und Geruch in diesen Fällen trotz der Verdünnung durch weitere Bestandteile wahrnehmbar bleiben soll. Die Wahrnehmbarkeitsschwelle von Diacetyl liegt bei etwa 0,1 ppm, und der Gehalt an Diacetyl liegt im erfindungsgemäßen Produkt nicht unter 1 ppm. Besonders bevorzugt sind ca. 10-20 ppm, und in manchen Fällen kann es gelingen, ihn noch weiter zu steigern.

Wie bereits voranstehend erwähnt, wird das erfindungsgemäße proteinhaltige Präparat aus einem pflanzlichen Ausgangsmaterial mit hohem Proteingehalt i.Tr. erhalten. Dieser Gehalt kann natürlicherweise vorhanden sein, oder aber das pflanzliche Ausgangsmaterial wird vorbehandelt, um diesen Gehalt zu erzielen. So sind geeignete pflanzliche Ausgangsmaterialien proteinreiche Pflanzenextrakte, die aus Lupinen erhältlich sind. Gängig sind, wie aus dem oben beschriebenen Stand der Technik bekannt, wässrige Pflanzenextrakte, deren Trockensubstanz zu etwa einem Drittel aus Fett, einem Drittel aus Protein und einem Drittel aus Kohlenhydraten besteht. Die Erfindung geht im Gegensatz hierzu von deutlich höheren Proteinanteilen in der Trockensubstanz aus, und dies führt überraschenderweise zu dem Effekt, dass im fermentierten Produkt ein "bohniges" Fehlaroma objektiv entweder gar nicht oder nur in deutlich verringerter Menge vorhanden ist und im letzteren Fall durch die Aromen, die als "milchproduktartig" charakterisiert werden können, so stark überdeckt wird, dass es subjektiv nicht wahrgenommen wird.

Ebenfalls überraschend war der Befund, dass die technofunktionellen Eigenschaften des erfindungsgemäßen proteinhaltigen Präparates denen des jeweils gewählten Ausgangsmaterialien vergleichbar sind, d.h. trotz der Fermentation keine Verschlechterung dieser Eigenschaften zu beobachten ist. Dies gewährleistet gute Emulgier- und Schaumbildungseigenschaften, wie sie für viele Anwendungen benötigt werden.

Das erfindungsgemäße proteinhaltige Präparat ist in der Regel völlig oder im wesentlichen lactosefrei, da die Ausgangsmaterialien in der Regel lactosefrei sind. In den meisten Fällen wird auch kein Anlaß bestehen, ihm Lactose zuzusetzen, obwohl dies in spezifischen Fällen ohne weiteres möglich ist. Außerdem ist es in der Regel völlig oder im wesentlichen cholesterinfrei, weil die pflanzlichen Ausgangsmaterialien im Gegensatz zu entsprechenden tierischen Materialien in der Regel kein Cholesterin enthalten. Und natürlich ist es in der Regel frei von tierischem Protein oder anderen tierischen Bestandteilen, es sei denn, diese werden aus speziellen Gründen zugesetzt.

Der Proteingehalt des erfindungsgemäßen Proteinpräparates ist gegenüber dem des eingesetzten Ausgangsmaterials im wesentlichen unverändert.

Je nach Wunsch kann das erfindungsgemäße proteinhaltige Präparat pasteurisiert oder anderweitig sterilisiert, also ein Präbioticum sein, oder es kann noch lebende Mikroorganismen enthalten, also ein biologisch aktives, probiotisches Lebensmittel oder eine solche Zutat für Lebensmittel sein. Ein probiotisches Lebensmittel wird vorzugsweise eingestellt auf einen Gehalt von 10⁶ bis 10¹², stärker bevorzugt von etwa 10⁸ bis 10¹⁰ und insbesondere von etwa 10⁹ Mikroorganismen pro Gramm Lebensmittel, sofern dieser Gehalt nicht bereits gegeben ist.

Das Präparat kann als Proteinlösung oder Proteindispersion erhalten und anschließend entweder in flüssiger oder getrockneter Form (beispielsweise sprühgetrocknet oder in vergleichbarer Weise konvektiv getrocknet) eingesetzt werden. Überraschenderweise wurde festgestellt, dass auch in getrockneten Produkten das milchproduktartige Aroma erhalten bleibt.

Das erfindungsgemäße proteinhaltige Präparat eignet sich beispielsweise als Lebensmittelzutat in
- joghurtartigen Produkten auf pflanzlicher Basis
- Joghurt
- milchartigen Drinks mit 0,1 - 3,5 % Fett auf pflanzlicher Basis
- aromatisierten Milchdrinks
- Eiscreme mit milchproduktartigem Aroma
- lactosefreier Eiscreme mit milchproduktartigem Aroma
- Dessertspeisen
- Reisprodukten (lactosefrei) mit milchproduktartigem Aroma
- Backmitteln
- feinen Backwaren

Die Herstellung des erfindungsgemäßen proteinhaltigen Präparats beginnt mit der Rohstoffvorbereitung. Dabei geht man von Lupinensamen als pflanzlichen Ausgangsmaterialien aus, weil diese in großem Umfang angebaut werden und sich aufgrund eines akzeptablen Proteingehaltes eignen.

Wie bereits erwähnt, wird bei Bedarf der Trockenmassen-Gehalt an Protein erhöht, wenn dieser im Rohmaterial nicht ausreichend hoch ist. So kann beispielsweise bei der Rohstoffvorbereitung eine Entölung (z.B. mit einem lipophilen Lösungsmittel wie Hexan oder mit CO₂) stattfinden. Außerdem können bei Bedarf Kohlenhydrate abgetrennt werden. Weitere Schritte, wie sie aus dem Stand der Technik bekannt sind, können natürlich ebenfalls vorgesehen sein, so z.B. ggf. eine Entbitterung der Ausgangsmaterialien oder dergleichen. Ein günstiges Ausgangsmaterial ist beispielsweise dasjenige, das man durch Behandlung von Lupinensamen gemäß EP 1 024 706 B1 erhält. Lupinensamen enthalten natürlicherweise etwa 38-50% der Trockenmasse Protein und damit etwas mehr als z.B. Soja oder gar Raps. Mit dem in der genannten EP-Patentschrift beschriebenen Behandlungsverfahren kann man sehr reine Proteinisolate erhalten. Solche sehr reinen Materialien; auch aus anderen pflanzlichen Proteinquellen, sind erfindungsgemäß sehr gut geeignet; es sollte aber klar sein, dass ein derart hoher Reinheitsgrad an Protein für die vorliegende Erfindung zwar besonders günstig, aber nicht Voraussetzung ist. Es kann beispielsweise genügen, die eingesetzten Pflanzenteile zu entölen und ggf. von enzymatischer Aktivität zu befreien, die einen negativen Einfluß haben könnte, und/oder zu entbittern. Ob das pflanzliche Ausgangsmaterial für die Fermentierung eine kleinere Menge an Mono-, Oligo- und/oder Polysacchariden enthalten soll oder nicht, wird der Fachmann in Anbetracht des angestrebten Verwendungszwecks entscheiden.

Das Rohmaterial wird bei der Rohstoffvorbereitung in eine für die Fermentation geeignete Form überführt, z.B. in eine wässrige Suspension oder Lösung. Der Fachmann kennt die hierfür notwendigen verfahrenstechnischen Schritte wie Zerkleinerung des Pflanzen-Ausgangsmaterials, Extraktion, Trennung von Proteinextrakt und Faserfraktion, Proteinfällung, Trocknung und dergleichen und wird sie im benötigen Umfang anwenden, z.B. im Rückgriff auf die oben erwähnte EP 1 024 706 B1.

Je nach Zusammensetzung der zu fermentierenden Suspension oder Lösung müssen oder können dieser bei Bedarf oder Wunsch Zusätze beigegeben werden. So ist darauf zu achten, dass eine ausreichende Menge an Zucker (z.B. Glucose) vorhanden ist, der den fermentierenden Mikroorganismen als Nährstoffquelle dient, und dieser muss oder kann ggf. zugesetzt werden, oder es müssen oder können Zusätze beigegeben werden, die während der Fermentation solche Zucker aus vorhandenen Kohlenhydraten freisetzen. Weiterhin muss den Mikroorganismen eine geeignete Stickstoffquelle zur Verfügung stehen. Wenn die zu fermentierende Suspension oder Lösung diese nicht in ausreichendem Maße bieten kann, beispielsweise durch vorhandene Aminosäuren, müssen entsprechende stickstoffhaltige Verbindungen oder Zusätze, die solche Verbindungen aus dem vorhandenen Material freisetzen, zugesetzt werden, wie im Stand der Technik bekannt. Als Stickstoffquelle eignet sich beispielsweise ein Hefeextrakt. Gleiches gilt für die Mineralsalze, deren Anwesenheit für die Stoffwechselaktivität der Mikroorganismen erforderlich ist. Auch sie können ggf. zugesetzt werden.

Die Fermentation wird in an sich bekannter Weise mit Hilfe von Milchsäure produzierenden Mikroorganismen durchgeführt. Die Fermentation kann anaerob oder in Gegenwart von Sauerstoff, homofermentativ oder heterofermentativ erfolgen. Demzufolge gibt es in der Auswahl der Bakterien prinzipiell keine Beschränkung, sofern sie Milchsäure und Diacetyl produzieren können und nicht toxisch sind. So können Lactococcen wie *Lactococcus lactis* oder Lactobacillen wie *Lactobacillus casei* eingesetzt werden, die beide L-Milchsäure produzieren, oder andere Bakterien wie *Pediococcus damnosus,* bei deren Einsatz man ein Milchsäure-Racemat erhält. Besonders günstig ist es, solche Bakterien für die Fermentation einzusetzen, die entweder reine L-Milchsäure erzeugen und/oder die in der Lage sind, schnell eine große Menge an Milchsäure zu produzieren. Es hat sich herausgestellt, dass unter diesem Aspekt insbesondere Lactobacillen der Stämme *Lactobacillus perolens, Lactobacillus paracasei* oder *Lactobacillus plantarum* geeignet sind. *Lactobacillus perolens* ist ein 1985 von Back aus Limonade isolierter Mikroorganismus, u.a. hinterlegt bei der Deutschen Sammlung von Mikroorganismen und Zellkulturen GmbH (DSMZ) in Braunschweig, Deutschland unter der Nr. 12744. Der Organismus wurde von Prof. Dr. Werner Back, am 23. Oktober 2002 bei der DSMZ unter der Nr. DSM 15255 nach Budapester Vertrag hinterlegt. Die Adresse der DSMZ lautet: D-38124 Braunschweig; Mascheroder Weg 1 b. Die anderen Mikroorganismen, *Lactobacillus paracasei* und *Lactobacillus plantarum,* sind seit larigem bekannt und können käuflich erworben werden, z.B. bei der DSZM in Braunschweig, Deutschland. Beispiele für dort hinterlegte Stämme sind z.B. DSZM 5622, 2649, 5457, 8741, 8742, 20006, 20020, 20207, 20244, 20312 oder 46331.

Für die Herstellung des Fermentationsmediums kann man übliche Methoden anwenden. So können die Zutaten mit Ausnahme von Glucose gemischt und ggf. mit Wasser verdünnt werden, z.B. durch Einbringen der Mischung in Wasser, das bereits im Fermenter vorgelegt wurde. Um das Wachstum fremder Mikroorganismen zu verhindern, sind geeignete Maßnahmen zu treffen, beispielsweise eine Pasteurisierung oder Tyndallisation des Fermentationsmediums. In geeigneter Weise kann dann dem Nährmedium nach dem Erhitzungsschritt eine für den gewählten Organismus verwertbare Kohlenstoffquelle wie z.B. Glucose zugesetzt werden. Damit werden Bräunungsreaktionen im Fermentationsmedium verhindert. Anschließend wird der Fermenter mit dem Inokulum des entsprechend gewählten Mikroorganismus beimpft. Geeignet ist beispielsweise eine etwa 1 %ige Bakteriensuspension. Alternativ können die Mikroorganismen natürlich auch auf einem stationären Substrat immobilisiert eingesetzt werden. Die Fermentation kann batchweise oder kontinuierlich erfolgen; dem Fachmann sind die geeigneten Maßnahmen hierfür bekannt, sie weichen vom Üblichen nicht ab. Die Fermentation erfolgt bei einer für das gewählte Bakterium geeigneten Temperatur. Der Kontakt mit dem Fermentationsmedium kann einige Stunden, ggf. auch einige Tage lang andauern, je nachdem wie schnell der Mikroorganismus Milchsäure produziert. Der absinkende pH-Wert kann ggf. abgepuffert werden, um das Medium möglichst lange in einem pH-Wert-Bereich zu halten, in dem weitere Milchsäure produziert wird. Mit dieser Maßnahme kann die Milchsäureproduktion auf deutlich über 10g/l gesteigert werden.

In einer bevorzugten Ausgestaltung der Erfindung wird dem Fermentationsmedium Fruchtsäure, vorzugsweise Citronensäure zugesetzt. Dadurch kann die Menge an Diacetyl gesteigert werden. Die Erfinder haben bei dieser Beeinflussung der Fermentation insbesondere im Fall von Citronensäure eine lineare Korrelation zwischen der Menge an zugesetzter Fruchtsäure und gebildetem Diacetyl feststellen können. Eine Menge von ca. 2g/l Citronensäure hat sich als günstig erwiesen. Der Fermentationsverlauf wird hierdurch nicht negativ beeinflußt.

Als Endprodukt erhält man eine Lösung oder eine Suspension, die je nach eingesetzter Konzentration der Lösung oder Suspension des Rohmaterials in der Regel etwa 5 bis 25% Trockenmasse, vorzugsweise etwa 15 bis 20% Trockenmasse besitzt. Der Diacetylgehalt liegt in der Regel bei etwa 9 bis 21 ppm. Antinutritiv wirkende α-glycosidisch verknüpfte Kohlenhydrate sind meist nicht oder nahezu nicht vorhanden.

Technologisch wichtige Parameter des erfindungsgemäßen Proteinpräparats können leicht in geeigneter Weise eingestellt werden. So konnte in einer 1 %igen Lösung des erfindungsgemäßen Proteinpräparates (ca 85 % Protein TS; Ausgangsmaterial ca. 95 % Protein TS), hergestellt nach dem voranstehend beschriebenen Verfahren, eine Emulgierfähigkeit (Emulgierkapazität) bei pH 7 im Bereich von 400 bis über 500 ml Öl/g Protein und in einer 10% igen Lösung eine Emulgieraktivität von 40-50% beobachtet werden, wobei die Kontrollgruppe (identisches Ausgangsmaterial, nicht fermentiert) unter denselben Bedingungen 500 ml Öl/g Protein emulgieren konnte. Die Testbedingungen für die Emulgierfähigkeit (Emulgierkapazität) sehen vor: 1) Herstellen der Proteindispersion/Lösung, 2) kontinuierliche Zugabe von pflanzlichem Öl unter Rühren und Emulgieren der Mischung (O/W) mit Laborreaktor (IKA LR-A1000 mit UltraTurrax T25 bei 11000 UpM.) und Bewerten des maximalen Volumens an Öl in ml bis zur Phaseninversion (=Emulgierkapazität). Handelsübliches Milcheiweiß (Na-Caseinat) besitzt unter vergleichbaren Testbedingungen eine Emulgierkapazität von 800-900 ml Öl/g Protein. Die Testbedingungen für die Emulgieraktivität sehen vor: 1) Herstellen der Proteindispersion/Lösung, 2) Mischung aus pflanzlichem Öl und dieser Lösung im verhältnis 1:1, 3) Rühren und Emulgieren der Mischung mit Laborrührer und 4) Zentrifugieren der Mischung/Emulsion (3000xg und 5 min.) und Bewerten des Volumens an Emulsionsphase in Prozent (=Emulgierakivität). Handelsübliches Milcheiweiß (Na-Caseinat) besitzt unter vergleichbaren Testbedingungen eine Emulgieraktivität von 90 %.

Bei erfindungsgemäßen Proteinpräparaten mit einem Feststoffgehalt von 8 bis 20% kann man bei pH 7 und nach 30-minütiger Wärmebehandlung bei 90 °C und 3-stündiger Lagerung bei 3 °C die Fähigkeit zur Bildung von Gelen mit meßbarer Festigkeit beobachten. Hierfür wurde als Meßgerät Stable Mirco Systems, TAX-T2, Surrey, GB verwendet.

Bei pH 7 lag die Schaumaktivität der erfindungsgemäßen Proteinpräparate bei mindestens 600% und vorzugsweise bei über 1000% bei einer Schaumdichte von 190 bis 250 g/l. Zum Vergleich: Das unbehandelte Ausgangsmaterial besaß eine Schaumaktivität von 900 bis 1200% und eine Schaumdichte von 150 bis 200 g/l. Als Aufschlagmaschine wurde eine Hobart 50-N verwendet. Hühnereiweißpulver (Eiklar) mit 12,6 % Trpckensubstanzanteil in Lösung besitzt unter den gleichen Testbedingungen nach 4 Minuten eine Schaumaktivität von 1500 % und eine Schaumdichte von 70g/l.

Das erfindungsgemäße Proteinpräparat kann entweder als solches oder aber als Lebensmittelzutat eingesetzt werden. Möglichkeiten hierfür sind oben aufgelistet. Die Verwendung in Speiseeis und die vorteilhaften Eigenschaften, die damit erzielt werden, sind in Beispiel 7 angegeben.

Die Erfindung soll nachstehend anhand von Ausführungsbeispielen näher erläutert werden.

### Beispiel 1

### Rohstoffvorbereitung

Lupinensaat wurde geschält und flockiert und anschließend gemäß EP 1 024 706 B1 entölt und entbittert. Bei einem pH-Wert, der in etwa dem isoelektrischen Punkt entspricht, wurden antinutritive Substanzen wie lösliche Kohlenhydrate abgetrennt. Das Protein des vorbehandelten Materials wurde extrahiert, indem es einem alkalischen Medium (pH 7-9) von 35 °C bis 45 °C ausgesetzt wurde, wobei eine Fraktionierung zwischen Raffinat und Proteinextrakten erfolgte. Aus dem Proteinextrakt wurde im sauren Medium (pH 4,5) eine Proteinfällung durchgeführt. Der resultierende "Proteinquark" wurde thermisch behandelt und einer Sprühtrocknung unterworfen. Das so erhaltene Proteinisolat besaß die folgende Zusammensetzung (Gew.-%):
Wasser 5-7
Trockenmasse 92-93
Rohproteingehalt (i.Tr.) >90%
Fettgehalt (i.Tr.) <2,5%
Kohlenhydrate (i.Tr.) <1%.

### Beispiel 2

### Fermentation mit Lactobacillus perolens anaerob

Das Proteinisolat aus Beispiel 1 wurde mit Hefeextrakt, Mineralsalz und Citronensäure gemischt (Zusammensetzung: 15% Proteinisolat, 0,5% Hefeextrakt, 0,5% Mineralsalze, 0,2% Citronensäure) und in zuvor sterilisiertem Wasser, das bereits im Fermenter vorgelegt wurde, dispergiert. Anschließend erfolgte eine Tyndallisierung des Fermentationsmediums:
1. Pasteurisation bei 72 °C für 10 min
2. Bebrütung des Mediums für 24h bei 30 °C
3. Pasteurisation bei 82 °C für 10 min
Nach Ende des 2. Pasteurisationsschritts erfolgte die Zugabe von D(+)-GlucoseMonohydrat in einer Menge von 2 Gew.-%, was bis zu diesem Zeitpunkt aufgeschoben wurde, um Bräunungsreaktionen im Fermenter zu verhindern. Anschließend wurde der Fermenter mit dem Inokulum des Mikroorganismus (1 % Bakteriensuspension bezogen auf den Fermenterinhalt) beimpft. Man ließ die Mischung 48 Stunden lang bei 27 °C anaerob fermentieren.

Als online-Messgrößen wurden erfasst: pH-Wert, Temperatur, Drehzahl des Fermenters, Gelöstsauerstoff. Als analytische Messgrößen wurden die Keimzahl, die Menge an Diacetyl als Aromaleitsubstanz und die Milchsäurekonzentration erfasst.

Nach 48h wurde die Fermentation durch eine 10-minütige Pasteurisation des Mediums bei 72 °C beendet.

Gehalt des Fermentationsmediums nach 48 h Fermentation:
L+Milchsäure 17,5 g/l; pH 4.1
Diacetyl 11,5 ppm

Das Beispiel wurde mit geänderten Mengen an Citronensäure (0g/5g) wiederholt, wobei festgestellt wurde, dass die Entstehung von Diacetyl linear mit der Menge an Citronensäure korreliert und den Fermentationsverlauf nicht negativ beeinflusst. Wurde keine Citronensäure zugesetzt, konnte im Endprodukt eine etwa um den Faktor 1,5-2 geringere Diacetylkonzentration nachgewiesen werden.

### Beispiel 3

### Fermentation mit Lactobacillus perolens aerob

Beispiel 2 wurde wiederholt, mit der Maßgabe, dass bei im übrigen unveränderten Bedingungen die Fermentation unter aeroben Bedingungen mit 15% Sauerstoffsättigung geführt wurde.
Gehalt des Fermentationsmediums nach 48 h Fermentation:
L + Milchsäure 17,5 g/l; pH 4.2
Diacetyl 21,9 ppm

### Beispiel 4

### Fermentation mit Lactobacillus paracasei anaerob

Die Fermentation wurde mit dem unter Beispiel 2 angeführten Fermentationsmedium und Fermentationsbedingungen, jedoch unter Verwendung von *Lb. paracasei* durchgeführt.

Milchsäure- und Diacetylgehalt nach 48 h Fermentation:
L + Milchsäure 13,9 g/l, pH 4.0
Diacetyl 2-3,8 ppm

### Beispiel 5

### Fermentation mit Lactobacillus paracasei aerob

Beispiel 4 wurde wiederholt, mit der Maßgabe einer aeroben Prozessführung, wie unter Beispiel 3 beschrieben. Nach 48 h Fermentation wurden folgende Gehalte gemessen:
L + Milchsäure 14,5 g/l, pH 4.0
Diacetyl 6,98 ppm

### Beispiel 6

### Herstellung von Speiseeis

Für die Eisherstellung wurden Proteinpräparate gemäß den Beispielen 2 bis 5 in sprühgetrockneter Form neben einem Standardpräparat (milcheiweißhaltig) und einem nicht fermentierten Präparat pflanzlichen Ursprungs eingesetzt. Verschiedene Eiscremerezepturen wurden untersucht.

Ein industrielles Verfahren zur Speiseeisbereitung wurde herangezogen und entsprechend auf den Labormaßstab zugeschnitten.

Die Zubereitung des Eismix erfolgt in einem beheizbaren Laborreaktor (IKA), welcher mit einem Mischer versehen ist. Um eine homogene Struktur zu erhalten, wird ein Rotor-Stator-System (Ultra-Turrax) verwendet. Im ersten Schritt wird das Wasser im IKA-Reaktor vorgelegt und auf 95 °C erhitzt. Die pulverförmigen Rezepturbestandteile werden abgewogen, gemischt und unter laufendem Rührwerk (ca. 100 U/min) sowie Homogenisator (IKA 8500 U/min) zudosiert. Daraufhin wird das Öl spontan zugegeben. Während des gesamten Vorgangs wird die Temperatur kontrolliert. Wenn der Mix eine Temperatur von 75 °C erreicht hat, wird für 2 Minuten weiter pasteurisiert. Anschließend wird vom Heizkreislauf auf den Kühlkreislauf umgeschaltet und der Mix wird unter laufendem Rührwerk und Homogenisator bis auf 15 °C abgekühlt. Der fertige Eismix wird abgefüllt und für 24 h bei 5°C reifen gelassen, damit die Aromakomponenten ihre Wirkung entfalten können. Mit einer Eismaschine mit Kälteakku und Rührwerk wird der Eismix ausgefroren und bei -20 °C für 24 h in einem Kühlraum gehärtet. Nach Ablauf dieser Zeit werden die Textur, das Abschmelzverhalten und die sensorischen Eigenschaften des Speiseeises charakterisiert.

In einer ersten Versuchsreihe wurde der Anteil an substituierten Milchproteinen variiert. Dabei wurden 0%, 25%, 50% und 100% der Milchproteine durch das Proteinpräparat gemäß Beispiel 2 substituiert. Die verwendeten Ausgangsmaterialien sind aus Tabelle 1 zu ersehen.

**Tabelle 1: Rezepturen für Typ Eiscremeprodukte mit Pflanzenfett und Substitutionsprodukte:**

| | | **Milcheis** | **Eiscreme auf pflanzlicher Basis** | | | |
|---|---|---|---|---|---|---|
| Zusammensetzung Nr. | | V | 1 | 2 | 3 | 4 |
| Substitutionsanteil Milchprotein | | Referenz | 10 % | 25 % | 50 % | 100 % |
| Wasser | ml | 320 | 320 | 320 | 320 | 320 |
| Zucker | g | 62 | 56,5 | 60 | 60 | 62 |
| Glucosesirup trocken | g | 23 | 17,5 | 17,5 | 20 | 23 |
| Maltodextrin | g | -- | -- | -- | 5,5 | 15 |
| Pflanzenfett | g | 40 | 40 | 40 | 40 | 40 |
| Magermilchpulver | g | 28 | 39,5 | 34 | 22 | -- |
| Molkenpulver | g | 23,5 | -- | -- | -- | -- |
| Proteinpräparat Bsp. 2 | g | -- | 1,5 | 3,5 | 7,5 | 15 |
| Stabilisator (Carragen) | g | 3,5 | 3,5 | 3,5 | 3,5 | 3,5 |
| Vanillinzucker | g | 8,5 | 8,5 | 8,5 | 8,5 | 8,5 |
| Summe Bestandteile | g | 508,5 | 487,0 | 487,0 | 487,0 | 487,0 |

In einer zweiten Versuchsreihe wurden vier Eiscremes mit den fermentierten Trockenprodukten der einzelnen Beispiele 2 bis 5 hergestellt, wobei in den Eiscremerezepturen jeweils 50 % der Milchproteine durch das erfindungsgemäße Proteinpräparat substituiert wurden.

Insgesamt wurden acht Eiscremes auf ihr sensorisches Verhalten hin geprüft, von denen ein Produkt zum Vergleich ohne pflanzliche Proteine und ein Produkt mit einem nativen, nicht fermentierten Lupinenproteinisolat gemäß Beispiel 1 erzeugt wurden. Vier Eiscremes stammten aus der oben erwähnten zweiten Versuchsreihe, in der jeweils 50 % der Milchproteine durch Proteinpräparate der Beispiele 2 bis 5 ersetzt waren. Die restlichen beiden Eiscremes wurden mit den Zusammensetzungen 2 und 4 gemäß Tabelle 1 unter Verwendung des Trockenprodukts gemäß Beispiel 2 hergestellt.

### Charakterisierung der Eiscreme-Eigenschaften

### (A) Sensorische Merkmale

Tabelle 2 zeigt die sensorischen Merkmale der erzeugten Eiscremes.

Die erzeugten Eiscremes wurden hinsichtlich Form, Aussehen, Farbe, Geruch, Geschmack und Konsistenz/Mundgefühl bewertet.

Die Eiscreme 01 (ohne pflanzliche Proteine) erscheint auf dem Löffel gebrochen, kantig und in der Farbe gräulich-weißlich. Das Eis 02 (mit Proteinisolat) ist in der Form auf dem Löffel gebrochen, aber nicht so kantig. Die Farbe ist gelblich bis bräunlich. Eiscremes 03 bis 08 sind in der Form gebrochen, allerdings ist die Farbe gelblich bis weißlich.

Die Eiscremes 01 und 02 unterscheiden sich im Geruch nur gering. Beide Produkte riechen mild, nach Vanille und leicht süßlich. Der Geruch des erfindungsgemäßen Proteinpräparats gibt den Eiscremes 03-08 eine aromatische, säuerliche und joghurtartige Note.

Die Eiscremes 01 und 02 unterscheiden sich im Geschmack dahingehend voneinander, dass der Zusatz von 50 % Lupinenprotein gemäß Beispiel 1 dem milchartigen Geschmack eine zusätzliche nussige Note verleiht. Innerhalb der Eiscremeprodukte, die mit dem erfindungsgemäßen Proteinpräparat hergestellt wurden, wurde im Rahmen der Prüfung von allen Prüfern das Eis 03 als besonders gut schmeckende Eiscreme bewertet. Es wurden die Merkmale milchartig, joghurtartig, Vanille, leicht säuerlich festgestellt. Aber auch alle anderen Eiscremeprodukte (03 bis 08) zeigten dieses milchartige Geschmacksprofil und eine gute Cremigkeit.

Durch den Einsatz des erfindungsgemäßen proteinhaltigen Präparats können, wie die oben dargestellten Ergebnisse zeigen, Eiscremes mit "Einfacheiscreme"-Rezepturen hergestellt werden, die aber in der Konsistenz höherwertigen Rezepturen gleichwertig sind. Die Konsistenzmerkmale der Eiscremes 03 bis 08 werden üblicherweise den Eissorten Cremeeis, Eiercremeeis oder Rahmeis zugeschrieben.

**Tabelle 2: Sensorik der Merkmale der Eiscremeprodukte**

| Eis | Pflanzenproteinpräparat | Form Aussehen Farbe | Geruch | Geschmack | Konsistenz/ Mundgefühl |
|---|---|---|---|---|---|
| 01 | 0 % | Gebrochen, kantig, grünlich, weißlich | mild, Vanille, süßlich | Milchartig, süß, Vanille, mehliger Nachgeschmack | fettig, klebrig, leimig |
| 02 | 50 % unfermentiert | gebrochen, gelblich, bräunlich | mild süßlich, Vanille | Milchartig, malzig, mehlig, nussig, heuig süßlich | weniger kalt als 01, cremig, fein, locker |
| 03 | 50 % Präparat gemäß Beispiel 2 | wie 02 gelblich, weißlich | aromatisch, säuerlich, Vanille, joghurtartig | Milchartig, joghurtartig, Vanille, säuerlich | weniger kalt als 01, sahnig, cremig, fein, locker |
| 04 | 50 % Präparat gemäß Beispiel 4 | wie 03 | wie 03 aber weniger säuerlich | wie 03 | wie 03 |
| 05 | 50 % Präparat gemäß Beispiel 3 | wie 03 | wie 03 süßlich | wie 03 | wie 03 |
| 06 | 50 % Präparat gemäß Beispiel 4 | wie 03 | wie 03, süßlich | wie 03 | wie 03 |
| 07 | 25 % Präparat gemäß Beispiel 2 | wie 03 | weniger intensiv als 03 | weniger intensiv als 03 | wie 03 |
| 08 | 100 % Präparat gemäß Beispiel 2 | wie 03 | wie 03, aber intensiver | wie 03 | wie 03, aber fester, zäher |

### (B) Texturmerkmale

Die folgende Tabelle 3 zeigt die Festigkeit und das Abschmelzverhalten der untersuchten Eiscremes.

**Tabelle 3: Technologische Eigenschaften der Eiscremeprodukte**

| Eis | Eingesetzte Proteine | Substitutionsanteil [%] | Textur [N/m²] | Abschmelzzeit [g/min] |
|---|---|---|---|---|
| 01 | Milchproteine | 0 | 2,04 | 0,44 |
| 02 | Unfermentiertes Pflanzenprotein | 50 | 3,84 | 0,33 |
| 03 | Protein Beispiel 2 | 50 | 4,74 | 0,31 |
| 04 | Protein Beispiel 4 | 50 | 4,70 | 0,33 |
| 05 | Protein Beispiel 3 | 50 | 4,94 | 0,33 |
| 06 | Protein Beispiel 5 | 50 | 4,83 | 0,35 |
| 07 | Protein Beispiel 2 | 25 | 3,78 | 0,37 |
| 08 | Protein Beispiel 2 | 100 | 4,93 | 0,32 |

Von der rheologischen Betrachtungsweise ausgehend, ergab sich durch die Substitution der Milchproteine durch pflanzliche proteinhaltige Präparate eine verbesserte Cremigkeit, Konsistenz und ein angenehmeres Mundgefühl. Dies drückt sich aus in einer höheren Festigkeit der Eiscremeprodukte mit diesen Proteinpräparaten, die auf ein verbessertes Gefüge und auf eine verbesserte Struktur und Verteilung der Bestandteile hindeutet. Ein signifikanter Einfluss auf das Abschmelzverhalten lässt sich ebenfalls erkennen. Die Abschmelzrate der Eisprodukte nimmt mit zunehmendem Anteil an Lupinenprotein deutlich ab (vgl. Tab. 3).

Bei der Variation der Konzentration an erfindungsgemäßem Protein zeigte sich insbesondere, dass mit zunehmendem Anteil an pflanzlichem Protein die Cremigkeit und Konsistenz des Eisproduktes verbessert wird. Beim Produkt mit 100 %-iger Substitution des Milcheiweiß resultierte dies in einer relativ festen Struktur, die aber nicht als unangenehm bezeichnet wurde.

Wie in den vorherigen Punkten bereits ausgeführt, lässt sich die Struktur der Eiscremeprodukte durch den Zusatz von pflanzlichen Proteinen eindeutig verbessern.

Aufgrund des in der Regel leguminosenartigen Geschmacks sind solche Produkte jedoch von geringerem Interesse. Erst durch den Einsatz der erfindungsgemäßen Proteinpräparate lassen sich Produkte erhalten, die ein milchartiges Aromaprofil aufweisen. In allen Tests zeigte sich, dass das mit Hilfe der erfindungsgemäßen Proteinpräparate hergestellte Eis nahezu oder vollständig frei von leguminosenartigem Geschmack war. Insbesondere die mit Lactobacillus perolens fermentieren Produkte zeigten ein völlig reines Aromaprofil, vergleichbar mit dem reiner Milchprodukte.

## Patentansprüche

1. Proteinpräparat mit
- mindestens 60% Protein pflanzlichen Ursprungs, bezogen auf das Trockengewicht,
- einem milchartigen Aroma, das einer Menge von mindestens 1 ppm Diacetyl entspricht, und
- einem Gehalt an Milchsäure,
**dadurch gekennzeichnet, dass** es unter Verwendung von Lupinensamen hergestellt ist.

2. Proteinpräparat nach Anspruch 1 mit
- mindestens 70% Protein pflanzlichen Ursprungs, bezogen auf das Trockengewicht,
- einem milchartigen Aroma, das einer Menge von mindestens 7 ppm Diacetyl entspricht, und
- einem Gehalt von mindestens 0,5 Gew.-% Milchsäure.

3. Proteinpräparat nach Anspruch 2 mit
- mindestens 85% Protein pflanzlichen Ursprungs, bezogen auf das Trockengewicht,
- einem milchartigen Aroma, das einer Menge von mindestens 15 ppm Diacetyl entspricht, und
- einem Gehalt von mindestens 1,0 Gew.-% Milchsäure.

4. Proteinpräparat nach einem der voranstehenden Ansprüche, **gekennzeichnet dadurch, dass** es sich bei der Milchsäure vorwiegend oder ausschließlich um L-Milchsäure handelt.

5. Proteinpräparat nach einem der voranstehenden Ansprüche, **gekennzeichnet dadurch, dass** es lactosefrei und cholesterinfrei ist.

6. Proteinpräparat nach einem der voranstehenden Ansprüche, **gekennzeichnet dadurch, dass** es probiotische Milchsäurebakterien enthält.

7. Proteinpräparat nach einem der voranstehenden Ansprüche, **gekennzeichnet dadurch, dass** es in 10 %iger Lösung bei pH 7 eine Emulgieraktivität von 40 bis 50% besitzt und/oder dass es in 1 %iger Lösung mindestens 400 ml, bevorzugt mindestens 500 ml Öl/g Protein emulgieren kann.

8. Proteinpräparat nach einem der voranstehenden Ansprüche, **gekennzeichnet dadurch, dass** es bei einem pH-Wert von 7 über eine Schaumaktivität von mindestens 600%, vorzugsweise von über 950% und/oder eine Schaumdichte von 190 bis 250 g/l verfügt.

9. Verfahren zum Herstellen eines Proteinpräparats nach einem der voranstehenden Ansprüche, wobei man ein in geeigneter Weise vorbehandeltes pflanzliches Ausgangsmaterial mit mindestens 60 Gew.-% Pflanzenprotein, bezogen auf das Trockengewicht des Materials, mit einem Milchsäure produzierenden Mikroorganismus in Gegenwart einer oder mehrerer für den Mikroorganismus notwendigen Nährstoff-, Stickstoff- und/oder Mineralstoffquelle(n) in an sich bekannter Weise fermentiert, **dadurch gekennzeichnet, dass** das pflanzliche Ausgangsmaterial aus Lupinensamen besteht oder diese enthält.

10. Verfahren nach Anspruch 9, **gekennzeichnet dadurch, dass** der Mikroorganismus ausgewählt ist unter vorzugsweise homofermentativen und potentiell heterofermentativen Mikroorganismen, ausgewählt unter Lactococcen, Lactobacillen und Pediococcen.

11. Verfahren nach Anspruch 10, **gekennzeichnet dadurch, dass** der Mikroorganismus ausgewählt ist unter *Lactobacillus perolens, Lactobacillus paracasei* und *Lactobacillus plantarum.*

12. Verfahren nach einem der Ansprüche 9 bis 11, **gekennzeichnet dadurch, dass** die Fermentation mit einer Lösung oder Dispersion des Pflanzenproteins in einer Konzentration von 5-25 % Trockenmasse, vorzugsweise 15 bis 20 % Trockenmasse, durchgeführt wird.

13. Verfahren nach einem der Ansprüche 9 bis 12, **dadurch gekennzeichnet, dass** die Fermentation in einem Medium erfolgt, dem Citronensäure in einer Menge von 0,1 bis 2,5 g/l, vorzugsweise von etwa 2 g/l, beigegeben wurde.

14. Verfahren nach einem der Ansprüche 9 bis 13, **gekennzeichnet dadurch, dass** die Fermentation in Gegenwart eines Puffers erfolgt, der das Absinken des pH-Wertes durch das Entstehen der Milchsäure abpuffert.

15. Proteinpräparat nach einem der Ansprüche 1 bis 8, worin das milchartige Aroma erhalten wurde durch die biotechnologische Behandlung eines vorwiegend oder ausschließlich pflanzlichen Ausgangsmaterials.

16. Proteinpräparat nach einem der Ansprüche 1 bis 8, erhalten durch ein Verfahren gemäß einem der Ansprüche 9 bis 14.

17. Verwendung eines Proteinpräparats nach einem der Ansprüche 1 bis 8, 15 und 16 als Lebensmittelzutat.

18. Verwendung eines Proteinpräparats nach Anspruch 6 als probiotisches Lebensmittel.

19. Verwendung eines Proteinpräparats nach Anspruch 17 zur Herstellung von Speiseeis.

## Claims

1. A protein preparation, comprising
- at least 60% protein of plant origin, based on the dry weight,
- a milk-like aroma which corresponds to an amount of at least 1 ppm of diacetyl, and
- a content of lactic acid,
**characterized in that** it has been prepared using lupine seeds.

2. The protein preparation as claimed in claim 1, comprising
- at least 70% protein of plant origin, based on the dry weight,
- a milk-like aroma which corresponds to an amount of at least 7 ppm of diacetyl, and
- a content of at least 0.5% by weight of lactic acid.

3. The protein preparation as claimed in claim 2, comprising
- at least 85% protein of plant origin, based on the dry weight,
- a milk-like aroma which corresponds to an amount of at least 15 ppm of diacetyl, and
- a content of at least 1.0% by weight of lactic acid.

4. The protein preparation as claimed in one of the preceding claims, **characterized in that** the lactic acid is predominantly or exclusively L-lactic acid.

5. The protein preparation as claimed in one of the preceding claims, **characterized in that** it is lactose-free and cholesterol-free.

6. The protein preparation as claimed in one of the preceding claims, **characterized in that** it comprises probiotic lactic acid bacteria.

7. The protein preparation as claimed in one of the preceding claims, **characterized in that,** in a 10% solution at pH 7, it has an emulsifying activity of 40 to 50%, and/or **in that**, in a 1% solution, it can emulsify at least 400 ml, preferably at least 500 ml, of oil/g of protein.

8. The protein preparation as claimed in one of the preceding claims, **characterized in that,** at a pH of 7, it has a foam activity of at least 600%, preferably greater than 950%, and/or a foam density of 190 to 250 g/l.

9. A method for producing a protein preparation according to any of the preceding claims, wherein a plant starting material pretreated in a suitable manner having at least 60% by weight of plant protein, based on the dry weight of the material, is fermented in a manner known per se using a microorganism which produces lactic acid in the presence of one or more nutrient source(s), nitrogen source(s) and/or mineral source(s) necessary for the microorganism, **characterized in that** the plant starting material consists of or contains lupine seed.

10. The method as claimed in claim 9, **characterized in that** the microorganism is selected under preferably homofermentative and potentially heterofermentative microorganisms, selected from lactococci, lactobacilli and pediococci.

11. The method as claimed in claim 10, **characterized in that** the microorganism is selected from *Lactobacillus perolens, Lactobacillus paracasei* and *Lactobacillus plantarum.*

12. The method as claimed in anyone of claims 9 to 11, **characterized in that** the fermentation is carried out using a solution or dispersion of the plant protein in a concentration of 5-25% dry matter, preferably 15 to 20% dry matter.

13. The method as claimed in one of claims 9 to 12, **characterized in that** the fermentation is performed in a medium to which citric acid has been added in an amount of 0.1 to 2.5 g/l, preferably of about 2 g/l.

14. The method as claimed in one of claims 9 to 13, **characterized in that** the fermentation is performed in the presence of a buffer which buffers the fall in pH due to the formation of lactic acid.

15. The protein preparation as claimed in one of claims 1 to 8, wherein the milk-like aroma was obtained by the biotechnological treatment of a predominantly or exclusively plant starting material.

16. The protein preparation as claimed in one of claims 1 to 8 obtained by a method as claimed in one of claims 9 to 14.

17. The use of a protein preparation as claimed in one of claims 1 to 8, 15 and 16 as food ingredient.

18. The use of a protein preparation as claimed in claim 6 as probiotic food.

19. The use of a protein preparation as claimed in claim 17 for producing ice cream.

## Revendications

1. Préparation protéique contenant
- au moins 60 % de protéine d'origine végétale, par rapport au poids à sec,
- un arôme laiteux qui correspond à une quantité d'au moins 1 ppm de diacétyle, et
- une teneur en acide lactique,
**caractérisée en ce qu'**elle est réalisée en utilisant des graines de lupin.

2. Préparation protéique selon la revendication 1, contenant
- au moins 70 % de protéine d'origine végétale, par rapport au poids à sec,
- un arôme laiteux qui correspond à une quantité d'au moins 7 ppm de diacétyle, et
- une teneur en acide lactique d'au moins 0,5 % en poids.

3. Préparation protéique selon la revendication 2, contenant
- au moins 85 % de protéine d'origine végétale, par rapport au poids à sec,
- un arôme laiteux qui correspond à une quantité d'au moins 15 ppm de diacétyle, et
- une teneur en acide lactique d'au moins 1,0 % en poids.

4. Préparation protéique selon l'une des revendications précédentes, **caractérisée en ce que** l'acide lactique est essentiellement ou exclusivement de l'acide lactique L.

5. Préparation protéique selon l'une des revendications précédentes, **caractérisée en ce qu'**elle est exempte de lactose et exempte de cholestérol.

6. Préparation protéique selon l'une des revendications précédentes, **caractérisée en ce qu'**elle contient des bactéries probiotiques d'acide lactique.

7. Préparation protéique selon l'une des revendications précédentes, **caractérisée en ce que** dans une solution à 10 %, à un pH 7, elle possède une activité d'émulsionnement de 40 à 50 % et/ou dans une solution à 1 % elle est apte de faire émulsionner au moins 400 ml, de préférence au moins 500 ml d'huile/gramme de protéine.

8. Préparation protéique selon l'une des revendications précédentes, **caractérisée en ce qu'**à une valeur pH de 7, elle dispose d'une activité de moussage d'au moins 600 %, de préférence de plus de 950 %, et/ou d'une densité de mousse de 190 à 250 g/l.

9. Procédé pour produire une préparation protéique selon l'une des revendications précédentes, dans lequel on fait fermenter de manière connue en soi un matériau de départ végétal prétraité de manière appropriée, présentant au moins 60 % en poids de protéine végétale par rapport au poids à sec du matériau, avec un microorganisme produisant de l'acide lactique, en présence d'une ou de plusieurs source(s) de substance nutritive, d'azote et/ou de substance minérale, nécessaire(s) pour le microorganisme, **caractérisé en ce que** le matériau de départ végétal est constitué de graines de lupin ou en contient.

10. Procédé selon la revendication 9, **caractérisé en ce que** le microorganisme est choisi de préférence parmi les microorganismes homo-fermentatifs et potentiellement hétéro-fermentatifs, choisis parmi Lactococcus, Lactobacillus et Pediococcus.

11. Procédé selon la revendication 10, **caractérisé en ce que** le microorganisme est choisi parmi Lactobacillus perolens, Lactobacillus paracasei et Lactobacillus plantarum.

12. Procédé selon l'une des revendications 9 à 11, **caractérisé en ce que** l'on réalise la fermentation avec une solution ou une dispersion de la protéine végétale en une concentration de 5 à 25 % de la matière sèche, de préférence de 15 à 20 % de la matière sèche.

13. Procédé selon l'une des revendications 9 à 12, **caractérisé en ce que** la fermentation s'effectue dans un milieu auquel on a ajouté de l'acide citrique en une quantité de 0,1 à 2,5 g/l, de préférence d'environ 2 g/l.

14. Procédé selon l'une des revendications 9 à 13, **caractérisé en ce que** la fermentation s'effectue en présence d'un tampon qui tamponne la baisse de la valeur pH par la formation de l'acide lactique.

15. Préparation protéique selon l'une des revendications 1 à 8, dans laquelle l'arôme laiteux a été obtenu par le traitement biotechnologique d'un matériau de départ essentiellement ou exclusivement végétal.

16. Préparation protéique selon l'une des revendications 1 à 8, obtenue par un procédé selon l'une des revendications 9 à 14.

17. Utilisation d'une préparation protéique selon l'une des revendications 1 à 8, 15 et 16 à titre d'ingrédient alimentaire.

18. Utilisation d'une préparation protéique selon la revendication 6 à titre de produit alimentaire probiotique.

19. Utilisation d'une préparation protéique selon la revendication 17 pour produire de la glace comestible.
